# EUROPEAN PATENT SPECIFICATION

(11) **EP 3 448 359 B1**
(45) Date of publication and mention of the grant of the patent: **07.06.2023**
(21) Application number: 17727405.7
(22) Date of filing: 28.04.2017
(51) Int. Cl.: A61K 9/00, A61K 9/127, A61K 31/573, A61K 31/661, A61P 27/02, A61P 27/06, A61P 27/12

(54) **LIPOSOMAL CORTICOSTEROIDS FOR TOPICAL INJECTION IN INFLAMED LESIONS OR AREAS**
LIPOSOMALE CORTICOSTEROIDE ZUR TOPISCHEN INJEKTION IN ENTZÜNDETE LÄSIONEN ODER BEREICHE
CORTICOSTÉROÏDES LIPOSOMALES POUR INJECTION TOPIQUE DANS DES ZONES DE LÉSIONS OU ENFLAMMÉES

(30) Priority: 29.04.2016 EP 16167818
(43) Date of publication of application: 06.03.2019
(73) Proprietor: Laurentia Holding B.V., 1411 RM Naarden (NL); Universiteit Utrecht Holding B.V., 3584 CS Utrecht (NL); Singapore Health Services Pte. Ltd., Singapore 168582 (SG)
(72) Inventor: METSELAAR, Josbert Maarten, 1411 PH Naarden (NL); WONG, Chee Wai, Singapore 258709 (SG); CZARNY, Bertrand Marcel Stanislas, 78440 Guitrancourt (FR); WONG, Tina Tzee Ling, Singapore, 259396 (SG); STORM, Gerrit, 3584 CG Utrecht (NL)
(74) Representative: V.O.
(86) International application number: PCT/NL2017/050273
(87) International publication number: WO 2017/188818

(56) References cited:
- US-A1- 2004 224 010
- US-A1- 2011 033 468

## Description

The invention relates to the field of medicine. More specifically the invention relates to topical treatment of an inflamed lesion or area, such as an inflamed eye.

Noninfectious anterior uveitis (AU) are a group of immune-related, sight-threatening inflammatory conditions that account for 60% of all cases of uveitis seen in eye centres. These patients contribute significantly to the clinical load. The incidence of uveitis varies from 14 to 52.4/100,000 globally, with an annual prevalence of 69.0 to 114.5 per 100 000 persons. Uveitis is the cause of up to 10% of legal blindness in the United States, or approximately 30 000 new cases of blindness per year. Locally, up to a hundred patients with uveitis are seen in the outpatient uveitis clinics of the Singapore National Eye Centre every week.

AU may run a relapsing and remitting clinical course. Sight threatening eye complications can occur with prolonged uncontrolled inflammation, such as cataract, glaucoma, and swelling of the central retina. These complications lead to blindness in up to 25% of patients.

Current treatment for uveitis in general and AU in particular includes intensive and frequent instillation of steroid eye drops, often over weeks to months, and for more severe or recalcitrant cases, injection of steroid into the tissue around the eye or into the eye itself. Although steroid eye drops are effective in the treatment, they have poor ocular penetration and require intensive, up to hourly instillation in the initial treatment period to achieve the desired effect. Their efficacy is further limited by rapid washout from the eye by tear drainage and inadequate eye drop instillation technique, not uncommon in patients with poor vision. These problems with eye drops often lead to poor compliance to treatment, persistent inflammation and sight threatening complications related to chronic inflammation. In addition, the untargeted delivery of steroids to uninflamed ocular tissue can result in steroid related side effects like cataract and glaucoma.

In view of the often poor retention of drugs at a diseased target site, and in view of the fact that a need for frequent administrations involves discomfort for a patient, drug delivery systems have been investigated and developed in the art that aim at slow release of encapsulated drug. Examples of such slow release drug delivery systems are polymer matrices and devices. In addition, slow release nanoparticles including liposomes have been described.

For instance, US 2011/0033468 describes a drug delivery system comprising 50-90% free therapeutic agent and phospholipid vehicles encapsulating the remainder of the therapeutic agent for treatment of the eye. The vehicles are administered intravitreal, i.e. within the vitreous body. This mode of administration serves to prolong the lifetime of the therapeutic agent in the eye, by forming a local depot. The vehicles are composed of phospholipids such as 2-dioleoyl-sn-glycero-3-phosphatidylcholine (DOPC), 1-palmitoyl-2-oleoyl-sn-glycero-3-phosphatidylcholine (POPC) and dioleylphosphatidylglycerol (DOPG) which contain unsaturated fatty acids. Such unsaturated lipids form fluid phospholipid membranes that allows leakage, resulting in slow release of the therapeutic agent into the vitreous body. A disadvantage of the methods of US 2011/0033468 is that inflammatory cells in the eye are not directly targeted.

US 2004/0224010 describes liposome-based formulations for enhanced ophthalmic drug delivery containing a therapeutic agent, in particular diclofenac. The liposomal formulations target and adhere to the corneal surface resulting in a drug reservoir within the eye. The liposomes contain cationic agents and/or mucoadhesive compounds in order to increase ocular residence time and enable sustained extracellular release of the therapeutic agent. The methods of US 2004/0224010 also have the disadvantage that inflammatory cells in the eye are not directly targeted.

It is an object of the present invention to overcome one or more of the limitations mentioned above by providing treatment by topical administration of corticosteroid-containing liposomes according to the invention to an inflamed lesion or area in a subject suffering from an inflammatory disorder. Said treatment preferably involves direct targeting of inflammatory cells.

Accordingly, the invention provides liposomes composed of non-charged vesicle-forming phospholipids, wherein said phospholipids comprise long-chain saturated fatty acids, said liposomes optionally including not more than 10 mole percent of negatively charged vesicle-forming lipids and/or not more than 10 mole percent of PEGylated lipids, said liposomes having a selected mean particle diameter in the size range of 40-200 nm and comprising a corticosteroid in water-soluble form, for use in a method for the treatment of an ophthalmic inflammatory disorder selected form of uveitis or conjunctivitis in a subject by subconjunctival administration at a dose of at most 5 mg corticosteroid, wherein said treatment has a treatment frequency of at most once per two weeks.

As explained in more detail herein below, some embodiments provide liposomes for use according to the invention, wherein said liposomes are composed of:
- non-charged vesicle-forming phospholipids, wherein said phospholipids contain long-chain saturated fatty acids, having an aliphatic tail of at least 13 carbon atoms and
- optionally, not more than 10 mole percent of negatively charged vesicle-forming lipids, and/or
- optionally, not more than 10 mole percent of PEGylated lipids.

Said liposomes comprise a corticosteroid in water-soluble form.

In preferred embodiments of the invention, liposomal drug delivery vehicles are used that do not act as slow release drug delivery systems. Instead, inflammatory cells are directly targeted with liposomes that contain non-charged vesicle-forming phospholipids with long-chain saturated fatty acids. These liposomal vehicles are stable *in vivo*, with a minimal (if any) leakage of corticosteroid over time before they reach inflammatory target cells. Uptake of the liposomes by inflammatory target cells and subsequent intracellular release of the corticosteroid leads to a therapeutic benefit. Hence, upon topical administration close to, or in, a pathological lesion, slow and selective uptake of liposomes according to the invention by inflammatory target cells occurs and the corticosteroid is released intracellularly to become effective.

Preferred embodiments of the invention therefore involve administration of corticosteroid-loaded liposomes according to the invention, comprising non-charged vesicle-forming phospholipids with long-chain saturated fatty acids, having an aliphatic tail of at least 13 carbon atoms, to a region where inflammatory target cells are present. The present invention provides the surprising insight that improved therapeutic results are obtained, and/or that a lower treatment frequency is required, as compared to topical administration of prior art slow release drug delivery systems such as for instance disclosed in US 2011/0033468 and US 2004/0224010.

In some preferred embodiments, liposomes according to the invention are for use in a method for the treatment of an ophthalmic inflammatory disorder selected form of uveitis or conjunctivitis in a subject by subconjunctival administration. Without being bound to theory, it is believed that, when injected in the subconjunctival space, the steroid-containing liposomes according to the invention are slowly taken up by inflammatory cells located in the subconjunctival space and, subsequently, a sustained intracellular release of corticosteroid is selectively achieved in these inflammatory cells. It has been demonstrated by the present inventors that a single subconjunctival injection of liposomal triamcinolone acetonide phosphate (TAP) according to the invention, or a single subconjunctival injection of liposomal prednisolone phosphate (PLP) according to the invention, can effectively suppress both the initial inflammation and attenuate the inflammatory response from recurrent AU over a one-month period. A single subconjunctival injection of liposomal TAP or PLP according to the invention was better than intensive 3 hourly steroid eyedrops instillation in the initial first week of treatment, and achieved similar efficacy over the next 3 weeks. As shown in the Examples, a single dose of subconjunctival liposomal TAP or liposomal PLP according to the invention surprisingly provided better acute results in treatment of uveitis as compared to intensive treatment with eye drops that contained prednisolone acetate in free form, and these single doses were as effective in the long term therapy in suppressing inflammation as the intensive eye drops treatment. Liposomal formulations according to the invention in particular act faster as compared to eye drops containing a corticosteroid in free form. Moreover, a single dose of the liposomal formulations is sufficient for effective treatment. This reduces discomfort for patients who would otherwise have to receive multiple administrations of the drug. The single dose liposomal formulations according to the invention further surprisingly provide a sustained clinical benefit that attenuate the inflammatory response even after a rechallenge. In addition, it is also shown in the Examples that administration of the liposomal formulations is less associated with the steroid related side-effects cataract and glaucoma. In conclusion, direct targeting of inflammatory target cells with corticosteroid-loaded liposomes according to the present invention, containing non-charged vesicle-forming phospholipids with long-chain saturated fatty acids, having an aliphatic tail of at least 13 carbon atoms, results in an improved therapeutic outcome, and/or a need of a lower treatment frequency, and/or less adverse side-effects, as compared to prior art topical administration protocols.

Preferred corticosteroid-containing liposomes according to the invention to be administered by subconjunctival administration are composed of:
- non-charged vesicle-forming phospholipids comprising long-chain saturated fatty acids, preferably selected from the group consisting of DiPaltmitoyl Phosphatidyl Choline (DPPC), Hydrogenated Soy Bean Phosphatidyl Choline (HSPC), DiStearoyl Phosphatidyl Choline (DSPC), Hydrogenated Egg Phosphatidyl Choline (HEPC) and combinations thereof, and
- optionally, not more than 10 mole percent of negatively charged vesicle-forming lipids and/or PEGylated lipids.

Said liposomes are to be administered by subconjunctival injection.

In ophthalmic inflammatory disorders such as uveitis, inflammatory cells are located in the subconjunctival space and subconjunctival administration thus directly targets the liposomes to the inflammatory cells. The fact that the liposomes are composed of phospholipids comprising long-chain saturated fatty acids, having an aliphatic tail of at least 13 carbon atoms, ensures that the liposomes are relatively rigid because of a high transition temperature of the lipid bilayer. As a result there is minimal leakage of corticosteroid from the liposomes before they are taken up by the target inflammatory cells. The corticosteroids are released once the liposomes are taken up by the inflammatory cells and are degraded intracellularly, most likely by the lysosomal degradation pathway. Hence, as a result of the administration route and the composition of the liposomes, the corticosteroids are released from the liposomes intracellularly, preferably in inflammatory cells residing in the subconjunctival space of the eye. This way, the liposomes are very effective in suppressing the inflammatory response in the eye.

As used herein, the term "long-chain fatty acid" (LCFA) refers to fatty acids with aliphatic tails of at least 13 carbon atoms. Preferably, such long-chain saturated fatty acids have at least 13 carbon atoms and at most 21 carbon atoms. A non-limiting example of a LCFA is palmitic acid. Fatty acids are "saturated" when they have no double bonds between the carbon atoms.

Liposomes composed of non-charged vesicle-forming phospholipids comprising long-chain saturated fatty acids, said liposomes optionally including not more than 10 mole percent of negatively charged vesicle-forming lipids and/or not more than 10 mole percent of PEGylated lipids, said liposomes having a selected mean particle diameter in the size range of 40-200 nm and comprising a corticosteroid in water-soluble form, are herein also referred to as "liposomes for use in a method according to the invention", or as "liposomes according to the invention", or as "liposomes as described herein".

A pharmaceutical composition comprising such liposomes is herein referred to as "a pharmaceutical composition for use in a method according to the invention" or "a pharmaceutical composition comprising liposomes as described herein".

As used herein, the term "subject" encompasses humans and animals, preferably mammals. Preferably, a subject is a mammal, more preferably a human.

The term "treatment" refers to inhibiting a disorder, in particular suppressing inflammation, i.e., halting or reducing its development or at least one clinical symptom thereof, and/or to relieving at least one clinical symptom of the disorder.

As used herein "inflammatory disorder" is also meant to encompass inflammatory diseases and inflammatory conditions.

Liposomes as described herein or a pharmaceutical composition comprising such liposomes are administered locally to an inflamed lesion or area of a subject, preferably a human, in need thereof in a method according to the invention. In some embodiments, treatment in accordance with the invention comprises topical injection in an inflamed lesion or area in said subject by subconjunctival administration, preferably by subconjunctival injection. "Topical injection" refers to local administration using a needle. In a method or use of the invention, the liposomes are locally administered, preferably by injection, into the inflamed lesion or area. For instance, not according to the invention, if the inflammatory disorder is arthritis, preferably osteoarthritis, the liposomes are preferably injected into inflamed joints, most preferably by intraarticular injection. If the inflammatory disorder is an ophthalmic inflammatory disorder, the liposomes are administered to the eye. In such case, the liposomes are preferably administered to the conjunctiva in order to directly target inflammatory cells. If the inflammatory disorder is an inflammatory skin disorder, not according to the invention, the liposomes are preferably injected into the inflamed skin. According to the invention, the inflamed region or area is an inflamed eye. The mode of administration is subconjunctival injection in case of ophthalmic inflammatory disorders such as uveitis and conjunctivitis. Subconjunctival injection refers to injection underneath the conjunctiva, which lines the inside of the eyelids and covers the sclera.

"Treatment frequency" as used herein refers to the frequency of administration of (a pharmaceutical composition comprising) liposomes for use in a method according to the invention. For instance, a treatment frequency of once per two weeks means that a pharmaceutical composition is administered to a patient once every two weeks, i.e. administration of two different doses is separated by approximately two weeks. In clinical practice, this may be two weeks plus or minus one or two days. A treatment frequency of at most once per two weeks indicates that a pharmaceutical composition is administered to a patient once every two weeks or less often, such as once every three weeks, or once every four weeks, or only once as a single dose treatment. Thus, with a treatment frequency of at most once per two weeks the time between two doses is at least two weeks, or a second dose is not administered at all. A sole and single administration of a pharmaceutical composition as described herein is also encompassed within the term "treatment frequency of at most once per two weeks". Preferably treatment in accordance with the invention is treatment with a treatment frequency of at most once per month, meaning that the time between two doses is at least one month, or a second dose is not administered at all. In a particularly preferred embodiment, treatment according to the invention involves the administration of a single dose of liposomes according to the invention, wherein said dose is at most 5 mg corticosteroid in water-soluble form.

In some embodiments, said dose is at most 2.5 mg, or at most 1.5 mg, or most 1 mg, or at most 0.5 mg corticosteroid in water-soluble form.

According to the present invention, the dose of a pharmaceutical composition comprising liposomes for use in a method according to the invention is at most 5 mg corticosteroid. A "dose of at most x mg corticosteroid" means at most x mg corticosteroid per time, i.e. per single administration of liposomes. E.g., a "dose of at most 10 mg corticosteroid" means at most 10 mg corticosteroid per time, i.e. per single administration of liposomes. Said dose is at most 5 mg corticosteroid.

In particular, if the disorder is an ophthalmic disorder or eye inflammation after surgery, the volume that can be administered is typically at most 0.5 ml. Since the maximal concentration of corticosteroid is typically 10 mg/ml, a volume of at most 0.5 ml typically contains at most 5 mg corticosteroid. Hence, if the disorder is an eye inflammation after surgery, the dose is at most 5 mg corticosteroid. In some embodiments, said corticosteroid is selected from the group consisting of prednisolone phosphate, dexamethasone phosphate, triamcinolone phosphate and combinations thereof.

Further, not according to the invention, if the disorder is an arthritic disease or an inflammatory skin disorder, the volume that can be administered is typically at most 2 ml. Hence, if the disorder is an arthritic disease or an inflammatory skin disorder, the dose is preferably at most 20 mg corticosteroid. In some embodiments, said dose is at most 15 mg corticosteroid. In some embodiments, said dose is at most 10 mg corticosteroid, preferably at most 7.5 mg corticosteroid, more preferably at most 5 mg corticosteroid.

Preferably, liposomes used in accordance with the invention are included in a pharmaceutical composition. The pharmaceutical composition is formulated for topical application to an inflamed lesion or area. Further provided is therefore a a use of liposomes composed of non-charged vesicle-forming phospholipids, wherein said phospholipids comprise long-chain saturated fatty acids, said liposomes optionally including not more than 10 mole percent of negatively charged vesicle-forming lipids and/or not more than 10 mole percent of PEGylated lipids, said liposomes having a selected mean particle diameter in the size range of 40-200 nm and comprising a corticosteroid in water-soluble form, for the preparation of a medicament for the treatment of an ophthalmic inflammatory disorder selected from uveitis or conjunctivitis in a subject by subconjunctival administration at a dose of at most 5 mg corticosteroid, wherein said treatment has a treatment frequency of at most once per two weeks. A pharmaceutical composition comprising liposomes according to the invention preferably further comprises a pharmaceutically acceptable carrier, diluent and/or excipient. Preferably, such pharmaceutical composition comprises the liposomes in a aqueous solution or hydrogel, such as hyaluronan.

Liposomes for use in a method according to the invention have a mean particle diameter of 40-200 nm as determined by Dynamic Light Scattering using Malvern DLS measurement laser equipment. Preferably the liposomes have a diameter of between 75 and 150 nm. The liposomes preferably have a rather low polydispersity index, i.e. of below 0.2, which means that the particle size distribution is narrow.

Liposomes for use according to the present invention typically comprise non-charged vesicle-forming lipids from the group of phospholipids, that can be either artificially synthesized or that originates from a natural source, optionally being artificially modified. Preferably said non-charged vesicle-forming lipids are partially or wholly synthetic. Phosphatidylcholines (PC), including those obtained from natural sources or those that are partially or wholly synthetic, are suitable for use in the present invention. As used herein, the term "partially synthetic or wholly synthetic vesicle-forming phospholipids" means at least one vesicle-forming phospholipid which has either been artificially made or which originates from a naturally occurring phospholipid, which has been artificially modified. Preferred phospholipids contain long-chain saturated fatty acids because they yield a bilayer with a relatively high transition temperature. As explained herein before, this has the advantage that there is minimal leakage of corticosteroid from the liposomes before they are taken up by the target inflammatory cells. After uptake of the liposomes by the target inflammatory cells the corticosteroids are released from the liposomes intracellularly, resulting in improved therapeutic results and/or a need of a lower treatment frequency, as compared to prior art slow release delivery vehicles.

In some embodiments, at least 80% of the phospholipids of the liposomes according to the present invention comprise long-chain saturated fatty acids. In more preferred embodiments, at least 85% of the phospholipids of the liposomes according to the present invention comprise long-chain saturated fatty acids. More preferably, at least 90% of the phospholipids of the liposomes according to the present invention comprise long-chain saturated fatty acids. More preferably, at least 95% of the phospholipids of the liposomes according to the present invention comprise long-chain saturated fatty acids. More preferably, at least 96% or at least 97% or at least 98% or at least 99% of the phospholipids of the liposomes according to the present invention comprise long-chain saturated fatty acids. The higher the percentage of long-chain saturated fatty acids-containing phospholipids, the higher the transition temperature of the lipid bilayer and the more rigid the liposomes will be. In some embodiments, all of the phospholipids of the liposomes according to the present invention contain long-chain saturated fatty acids.

Some embodiments therefore provide liposomes composed of non-charged vesicle-forming phospholipids, wherein the fatty acids of at least 80% of said phospholipids are long-chain saturated fatty acids, said liposomes optionally including not more than 10 mole percent of negatively charged vesicle-forming lipids and/or not more than 10 mole percent of PEGylated lipids, said liposomes having a selected mean particle diameter in the size range of 40-200 nm and comprising a corticosteroid in water-soluble form, for use in a method for the treatment of an ophthalmic inflammatory disorder in a subject by subconjunctival administration at a dose of at most 5 mg corticosteroid, wherein said treatment has a treatment frequency of at most once per two weeks. Preferably, the fatty acids of at least 85% of said phospholipids are long-chain saturated fatty acids. More preferably, the fatty acids of at least 90% of said phospholipids are long-chain saturated fatty acids. More preferably, the fatty acids of at least 95% of said phospholipids are long-chain saturated fatty acids. More preferably, the fatty acids of at least 96% of said phospholipids are long-chain saturated fatty acids. More preferably, the fatty acids of at least 97% of said phospholipids are long-chain saturated fatty acids. More preferably, the fatty acids of at least 98% of said phospholipids are long-chain saturated fatty acids. More preferably, the fatty acids of at least 99% of said phospholipids are long-chain saturated fatty acids.

Further provided is a method for the treatment of an ophthalmic inflammatory disorder in a subject in need thereof, the method comprising administering to the subject by subconjunctival administration liposomes composed of non-charged vesicle-forming phospholipids, wherein the fatty acids of at least 80% of said phospholipids are long-chain saturated fatty acids, said liposomes optionally including not more than 10 mole percent of negatively charged vesicle-forming lipids and/or not more than 10 mole percent of PEGylated lipids, said liposomes having a selected mean particle diameter in the size range of 40-200 nm and comprising a corticosteroid in water-soluble form, at a dose of at most 5 mg corticosteroid, wherein said treatment has a treatment frequency of at most once per two weeks.

Further provided is a use of liposomes composed of non-charged vesicle-forming phospholipids, wherein the fatty acids of at least 80% of said phospholipids are long-chain saturated fatty acids, said liposomes optionally including not more than 10 mole percent of negatively charged vesicle-forming lipids and/or not more than 10 mole percent of PEGylated lipids, said liposomes having a selected mean particle diameter in the size range of 40-200 nm and comprising a corticosteroid in water-soluble form, for the preparation of a medicament for the treatment of an ophthalmic inflammatory disorder in a subject by subconjunctival administration at a dose of at most 5 mg corticosteroid, wherein said treatment has a treatment frequency of at most once per two weeks. As stated above, preferably the fatty acids of at least 85% of said phospholipids are long-chain saturated fatty acids. More preferably, the fatty acids of at least 90% of said phospholipids are long-chain saturated fatty acids. More preferably, the fatty acids of at least 95% of said phospholipids are long-chain saturated fatty acids. More preferably, the fatty acids of at least 96% of said phospholipids are long-chain saturated fatty acids. More preferably, the fatty acids of at least 97% of said phospholipids are long-chain saturated fatty acids. More preferably, the fatty acids of at least 98% of said phospholipids are long-chain saturated fatty acids. More preferably, the fatty acids of at least 99% of said phospholipids are long-chain saturated fatty acids.

The invention provides liposomes composed of non-charged vesicle-forming phospholipids, wherein the fatty acids of said phospholipids are long-chain saturated fatty acids, said liposomes optionally including not more than 10 mole percent of negatively charged vesicle-forming lipids and/or not more than 10 mole percent of PEGylated lipids, said liposomes having a selected mean particle diameter in the size range of 40-200 nm and comprising a corticosteroid in water-soluble form, for use in a method for the treatment of an ophthalmic inflammatory disorder selected from uveitis or conjunctivitis in a subject by subconjunctival administration at a dose of at most 5 mg corticosteroid, wherein said treatment has a treatment frequency of at most once per two weeks.

Further provided is a use of liposomes composed of non-charged vesicle-forming phospholipids, wherein the fatty acids of said phospholipids are long-chain saturated fatty acids, said liposomes optionally including not more than 10 mole percent of negatively charged vesicle-forming lipids and/or not more than 10 mole percent of PEGylated lipids, said liposomes having a selected mean particle diameter in the size range of 40-200 nm and comprising a corticosteroid in water-soluble form, for the preparation of a medicament for the treatment of an ophthalmic inflammatory disorder selected from uveitis or conjunctivitis in a subject by subconjunctival administration at a dose of at most 5 mg corticosteroid, wherein said treatment has a treatment frequency of at most once per two weeks.

Particularly preferred phospholipids are DiPaltmitoyl Phosphatidyl Choline (DPPC), Hydrogenated Soy Bean Phosphatidyl Choline (HSPC), DiStearoyl Phosphatidyl Choline (DSPC), and Hydrogenated Egg Phosphatidyl Choline (HEPC). Liposomes for use in a method according to the present invention comprise at most 10 mole % PEGylated lipids and/or at most 10 mole % of negatively charged lipids. Preferred PEGylated lipids are composed of a PEG polymer with a molecular mass between 200 and 20 000 dalton on the one end and a lipophilic anchoring molecule on the other end. Typically anchoring molecules are chosen from the group of phospholipids and sterols. Preferred PEGylated lipids are PEG 2000-DiStearoyl Phosphatidyl Ethanolamine (PEG-DSPE) and PEG 2000-cholesterol. Preferred negatively charged lipids are DiPalmitoyl Phosphatidyl Glycerol (DPPG) and DiStearoyl Phosphatidyl Glycerol (DSPG). Liposomes for use in a method according to the present invention further preferably comprise a sterol or steroid alcohol of synthetic or natural origin which have a hydroxyl group in the 3-position of the A-ring. Of this group of sterol compounds cholesterol is preferred.

The fraction of polymer lipid conjugates and negatively charged lipids is 0-10 mol%, and preferably 1 - 10 mol%, more preferably 2.5 - 10 mol%, based upon the total molar ratio of the vesicle-forming lipids in the formulation. If negatively charged lipids and especially polymer-lipid-conjugates are present in the liposomal formulation, the formulation will be physically stabilized. However, by carefully selecting specific lipid compositions at physical specifications, physical stability can be obtained without using a PEG-lipid-conjugate or negatively charged lipids. For example, 50-100 nm liposomes of DSPC and cholesterol and/or sphingolipids like sphingomyelin are suitable for use in a method according to the invention.

In a particularly preferred embodiment, the invention provides a liposome for use according to the invention or a method according to the invention, wherein said liposome comprises:
- 0-50 mol% of cholesterol,
- 50-90 mol% of non-charged partially synthetic or wholly synthetic vesicle-forming lipids, which are preferably non-charged vesicle-forming phospholipids with long-chain saturated fatty acids,
- 0-10 mol% of amphipatic vesicle-forming lipids coupled to polyethylene glycol, and
- 0-10 mol% of a negatively charged vesicle-forming lipid.

Such liposome is for instance made in accordance with the methods described in WO 02/45688 or WO 03/105805. However, doses and treatment frequencies and embodiments in accordance with the present invention are not described therein. Liposomes for use in a method according to the present invention preferably have a mean particle diameter size range of between about 75 and 150 nm. As stated before, said partially synthetic or wholly synthetic vesicle-forming lipid is preferably selected from the group consisting of DSPC, DPPC, HSPC, HEPC and combinations thereof.

Specific examples of liposomes for use in a method according to the invention are:
- liposomes composed of non-charged vesicle-forming lipids (preferably non-charged vesicle-forming phospholipids wherein the fatty acids of at least 90% of said phospholipids are long-chain saturated fatty acids), including up to 10 mole percent of an amphipathic vesicle-forming lipid derivatised with polyethylene glycol and optionally including not more than 10 mole percent of negatively charged vesicle-forming lipids, which liposomes have a selected mean particle diameter in the size range of 40-200 nm and contain a corticosteroid, characterised in that the corticosteroid is present in a water-soluble form;
- liposomes composed of cholesterol and non-charged vesicle-forming phospholipids selected from the group consisting of DSPC, HSPC, HEPC, DPPC and combinations thereof, which liposomes have a selected mean particle diameter in the size range of 40-200 nm and contain a corticosteroid, characterised in that the corticosteroid is present in a water-soluble form;
- liposomes composed of non-charged vesicle-forming phospholipids selected from the group consisting of DSPC, HSPC, HEPC, DPPC and combinations thereof, which liposomes have a selected mean particle diameter in the size range of 40-200 nm and contain a corticosteroid in water-soluble form;
- liposomes composed of non-charged vesicle-forming lipids (preferably non-charged vesicle-forming phospholipids wherein the fatty acids of at least 90% of said phospholipids are long-chain saturated fatty acids) and not more than 5 mole percent of negatively charged dipalmitoyl phosphatidyl glycerol, which liposomes have a selected mean particle diameter in the size range of 40-200 nm and contain a corticosteroid, characterised in that the corticosteroid is present in a water-soluble form;
- liposomes composed of cholesterol and non-charged vesicle-forming lipids selected from phospholipids that are partially or wholly synthetic (preferably non-charged vesicle-forming phospholipids wherein the fatty acids of at least 90% of said phospholipids are long-chain saturated fatty acids), optionally including not more than 5 mole percent of negatively charged vesicle-forming lipids, which liposomes have a selected mean particle diameter in the size range of 40-200 nm and contain a corticosteroid, characterised in that the corticosteroid is present in a water-soluble form.

As said, liposomes used in accordance with the present invention may be prepared according to methods used in the preparation of conventional liposomes or PEG-liposomes, for instance such as disclosed in WO 02/45688 or WO 03/105805. Passive loading of the active ingredients into liposomes by dissolving the water-soluble corticosteroids in the aqueous phase is sufficient in order to reach sufficient encapsulation, but other methods can also be used, so as to further increase the encapsulation efficiency. The lipid components used in forming the liposomes may be selected from a variety of vesicle-forming lipids, such as phospholipids, sphingolipids and sterols. Substitution (complete or partial) of these basic components by e.g. sphingomyelins and ergosterol is possible. For effective encapsulation of the water-soluble corticosteroids in liposomes, thereby avoiding leakage of the drug from the liposomes, especially phospholipid components having saturated, rigidifying acyl chains have appeared to be useful.

In some embodiments, the present invention encompasses the use of liposomes that are mainly composed of non-charged vesicle-forming phospholipids wherein the fatty acids of said phospholipids are long-chain saturated fatty acids, and wherein said liposomes also comprise a minor amount of phospholipids with unsaturated fatty acids, as long as these liposomes maintain their *in vivo* stability ensuring a minimal leakage of corticosteroid from the liposomes before the liposomes are taken up by the target inflammatory cells. The percentage of phospholipids with unsaturated fatty acids (based on the total amount of phospholipids in these liposomes) is at most 20%, preferably at most 15%, more preferably at most 10%, more preferably at most 8%, more preferably at most 5%, more preferably at most 4%, more preferably at most 3%, more preferably at most 2%, and more preferably at most 1%. As stated herein before, the higher the percentage of long-chain saturated fatty acids, the higher the transition temperature of the lipid bilayer and the more rigid the liposomes will be.

A liposomal composition for use in a method according to the present invention comprises a corticosteroid in water-soluble form, which is also referred to herein as a water-soluble corticosteroid. Such water-soluble corticosteroids encompass corticosteroids that are naturally soluble in water, as well as water-soluble corticosteroid derivatives. The term "water-soluble" is defined herein as having a solubility at a temperature of 25°C of at least 10 g/l water or water buffered at neutral pH. Water-soluble corticosteroids which can be advantageously used in accordance with the present invention are alkali metal and ammonium salts prepared from corticosteroids, having a free hydroxyl group, and organic acids, such as (C2 - C12) aliphatic, saturated and unsaturated dicarbonic acids, and inorganic acids, such as phosphoric acid and sulphuric acid. As alkaline metal salts the potassium and sodium salts are preferred. "Corticosteroid derivative" as used herein refers to a corticosteroid or derivative thereof. Such derivative is a chemically modified corticosteroid, preferably by esterification. Preferred corticosteroid derivatives in accordance with the invention are water-soluble phosphate, succinate and sulphate esters of a corticosteroid. Also other water-soluble, positively or negatively charged, derivatives of corticosteroids can be used. Non-limiting examples of water-soluble corticosteroid derivatives that can be applied in accordance with the invention are betamethasone sodium phosphate, desonide sodium phosphate, dexamethasone sodium phosphate, hydrocortisone sodium phosphate, hydrocortisone sodium succinate, methylprednisolone disodium phosphate, methylprednisolone sodium succinate, prednisolone sodium phosphate, prednisolone sodium succinate, prednisolamate hydrochloride, prednisone disodium phosphate, prednisone sodium succinate, triamcinolone acetonide disodium phosphate. Most preferred are prednisolone sodium phosphate, dexamethasone sodium phosphate and triamcinolone acetonide sodium phosphate.

Examples of inflammatory disorders that can be successfully treated with the liposomal compositions in accordance with the present invention are inflammatory disorders that are characterized by local inflammatory lesions or areas. Examples of such disorders are an inflammatory ophthalmic disorder such macular oedema, an arthritic disease, such as osteoarthritis, eye inflammation after surgery, such as cataract surgery or retina detachment surgery, and an inflammatory skin disorder, such as psoriasis and atopic dermatitis. Therefore, in a preferred embodiment the inflammatory disorder is selected from the group consisting of an inflammatory ophthalmic disorder, an arthritic disorder, eye inflammation after surgery, and an inflammatory skin disorder, more preferably said disorder is selected from the group consisting of uveitis, macular oedema, conjunctivitis, osteoarthritis, eye inflammation after cataract surgery or retina detachment surgery, psoriasis and atopic dermatitis. Preferably an inflammatory disorder that can treated with a method according to the present invention is an arthritic disease, preferably osteoarthritis or an inflammatory ophthalmic disorder. The inflammatory disorder that is treated with a method according to the present invention is uveitis or conjunctivitis, most preferably uveitis. "Uveitis" as used herein includes all types of uveitis, including anterior uveitis, intermediate uveitis and posterior uveitis. In a preferred embodiment, said uveitis is anterior uveitis.

Features may be described herein as part of the same or separate aspects or embodiments of the present invention for the purpose of clarity and a concise description. It will be appreciated by the skilled person that the scope of the invention may include embodiments having combinations of all or some of the features described herein as part of the same or separate embodiments.

The invention will be explained in more detail in the following examples.

### Brief description of the drawings

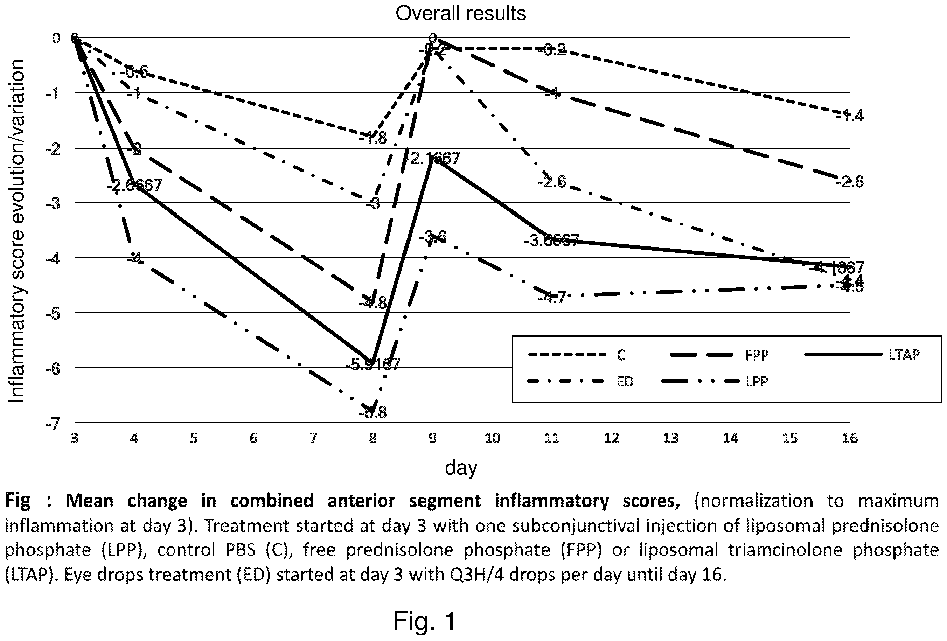

Figure 1: Mean change in combined anterior segment inflammatory scores (normalization to maximum inflammation at day 3).
   Treatment started at day 3 with one subconjunctival injection of liposomal prednisolone phosphate (LPP), control PBS (C), free prednisolone phosphate (FPP) or liposomal triamcinolone phosphate (LTAP). Eye drops treatment (ED) started at day 3 with Q3H / 4 drops per day until day 16.
Figure 2: Slit lamp and fundus photos at Day 11. Control eye had greater iris congestion, anterior chamber cells and flare than the eye treated with liposomal TA. Vitreous haze was slightly worse in the control eye.
Figure 3: Histology. Difference between control eyes (inflamed) and treated eyes is visible with HE staining and confirmed by immunostaining.
Figure 4: Cataract formation in the different treatment groups and control group.
   LPP: liposomal prednisolone phosphate
   LTAP: liposomal triamcinolone phosphate
   FPP: free prednisolone phosphate
   EDN: eye drop treatment with topical Predforte1% Q3H in non inflamed eye
   ED: eye drop treatment with topical Predforte1% Q3H in inflamed eye
   Control: PBS
Figure 5: Intraocular pressure (IOP) in the different treatment groups and control group.
   LPP: liposomal prednisolone phosphate
   LTAP: liposomal triamcinolone phosphate
   FPP: free prednisolone phosphate
   EDN: eye drop treatment with topical Predforte1% Q3H in non inflamed eye
   ED: eye drop treatment with topical Predforte1% Q3H in inflamed eye
   C: control PBS

### Examples

### Methods

### Pre immunization

A subcutaneous injection of *Mycobacterium tuberculosis* H37Ra antigen (10mg; Difco, Detroit, MI) suspended in mineral oil (500uL) was given as preimmunization (Ghosn et al., 2011). One week later, a second injection of the same amount of subcutaneous antigen was given at a separate site. Successful preimmunization was confirmed after one week by the presence of a visible skin nodule at the injection site.

### Induction of experimental uveitis

Experimental uveitis was induced by unilateral intravitreal injection on Day 0 in preimmunized rabbits (7 days after the second preimmunization). The rabbits were anesthetized with intraperitoneal injection of ketamine hydrochloride (35-50mg/kg) and Xylazil (5- 10mg/kg). Following topical anesthesia with Amethocaine 1%, the right eye of each rabbit was disinfected with 5% povidone iodine. An intravitreal injection of *Mycobacterium tuberculosis* H37Ra antigen suspended in sterile saline (50ug; 1ug/uL) using a Hamilton syringe with a 31-gauge needle was given through the superotemporal sclera, 1.5mm from the limbus. One drop of Tobramycin was instilled at the end of the procedure. To simulate a recurrence of uveitis, we induced experimental uveitis again at Day 8, following the procedure as described above. The eyes were clinically monitored for 30 days and graded for ocular inflammation by 2 masked investigators.

### Intervention

Rabbits were randomized into 4 intervention groups, 3 days after uveitis induction: one group received a single dose of 0.1ml subconjunctivally injected liposomal triamcinolone acetonide sodium phosphate (LTAP) (4mg/ml) (n=6), one group received a single dose of 0.1ml subconjunctivally injected liposomal prednisolone sodium phosphate (LPP) (4mg/ml) (n=5), one group received a single dose of 0.1ml subconjunctivally injected prednisolone phosphate (FPP) (4mg/ml), and one group received eye drop treatment with topical Predforte1% Q3H; 4 drops per day were administered for 2 weeks (ED) (n=5). A fifth group received a single dose of subconjunctivally injected PBS (control group (C); no treatment) (n=5).

The liposomal nanoparticles were composed of dipalmitoyl phosphatidyl choline (DPPC), cholesterol, and PEG2000 distearoyl phosphatidylethanolamine (PEG-DSPE) in a 62%, 33%, and 5% molar ratio (for liposomal nanoparticle preparation: see page 1134 of Lobatto et al., 2015).

Prior to injection, rabbits were anesthetized with intraperitoneal injection of ketamine hydrochloride (35-50 mg/kg) and Xylazil (5-10 mg/kg). Following topical anesthesia with Amethocaine 1%, the right eye of each rabbit was disinfected with 5% povidone iodine. A Hamilton syringe with a 31-gauge needle was used to deliver a single injection of 0.1ml of treatment. Topical Tobramycin was administered 4 times a day for 5 days after injection of subconjunctival steroid.

### Clinical Examination

Clinical examination was performed by 2 masked independent investigators. Slit-lamp biomicroscopy, measurement of intraocular pressure with the Tonopen, photography of the anterior segment and dilated fundal examination with binocular indirect ophthalmoscopy using a 20D lens were performed prior to uveitis induction and at 8 defined time points thereafter (Days 0, 1, 3, 4, 8, 9, 11, 16, 24 and 31). Clinical severity of uveitis was scored by evaluating anterior chamber cells/flares, vitreous haze, and iris vessels. These clinical scoring systems had been described in previous literature (Nussenblatt et al., 1985; Bloch-Michel & Nussenblatt, 1987). The combined anterior segment inflammatory score was defined as the sum of the scores for iris vessels, anterior chamber cells and anterior chamber flare. The presence of cataract was determined on slit lamp biomicroscopy on day 31 and graded based on the LOCS scale.

### Experimental schedule:

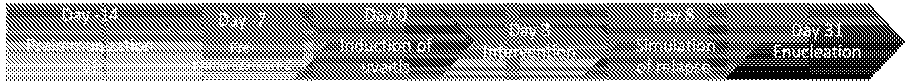

### Enucleation, euthanasia & pathology procedures

All rabbits were euthanized at the end of the study period of 30 days. Euthanasia was carried out with intraperitoneal pentobarbitone (60-150 mg/kg) followed by enucleation of the operated eyes.

### Histopathology and Immunohistochemistry

Eyes were embedded in paraffin or directly frozen in the case of the fluorescent liposomes.

For paraffin, whole rabbit eye was enucleated and fixed in 10% neutral buffered formalin solution (Leica Surgipath, Leica Biosystems Richmond, Inc.) for 24 hours. The whole rabbit eye was then dissected to anterior and posterior segment prior to dehydration in increasing concentration of ethanol, clearance in xylene, and embedding in paraffin (Leica-Surgipath, Leica Biosystems Richmond, Inc.) Five-micron sections were cut with a rotary microtome (RM2255, Leica Biosystems Nussloch GmbH, Germany) and collected on POLYSINE^{™} microscope glass slides (Gerhard Menzel, Thermo Fisher Scientific, Newington, CT). The sections were dried in an oven of 37°C for at least 24 hours. To prepare the sections for histopathological and immunohistochemical examination, the sections were heated on a 60°C heat plate, deparaffinized in xylene and rehydrated in decreasing concentration of ethanol.

For frozen eyes, whole rabbit eye was embedded in Optimal Cutting Temperature (OCT) compound at -20 _{°}C for 1 hour. Six-micron sections were cut with a cryostat (HM550, Thermo Fisher Scientific Microm International GmbH, Germany) and collected on POLYSINE^{™} microscope glass slides (Gerhard Menzel, Thermo Fisher Scientific, Newington, CT). Sections were air dried at room temperature (RT) for 1 hour.

A standard procedure for Hematoxylin and Eosin (H&E) was performed. A light microscope (Axioplan 2; Carl Zeiss Meditec GmbH, Oberkochen, Germany) was used to examine the slides and images were captured.

In parallel, immunofluorescence staining was performed. For paraffin, heat-induced antigen retrieval was performed by incubating sections in sodium citrate buffer (10 mM Sodium citrate, 0.05% Tween 20, pH 6.0) for 20 minutes at 95-100_{°}C. The sections were then cooled down in sodium citrate buffer for 20 minutes in RT and washed three times for 5 minutes each with 1X PBS. For frozen samples, the sections were fixed in 4% paraformaldehyde (PFA) in 1X Phosphate Buffered Saline (PBS) for 10 minutes and washed three times for 5 minutes each with 1X PBS.

Non-specific sites were blocked with blocking solution of 5% bovine serum albumin (BSA) in 0.1% Triton X-100 and 1XPBS for 1 hour at room temperature in a humidified chamber. The slides were then rinsed briefly with 1X PBS. A specific primary antibody shown in Table 1 was applied and incubated overnight at 4_{°}C in a humidified chamber prepared in blocking solution. After washing twice with 1X PBS and once with 1X PBS with 0.1% Tween for 10 minutes each, Alexa Fluro^{®} 488/594 - conjugated fluorescein-labelled goat anti-rabbit IgG secondary antibody (Invitrogen- Molecular Probes, Eugene, OR) was applied at a concentration of 1:1000 in blocking solution and incubated for 90 minutes at room temperature (RT).

The slides were then washed twice with 1X PBS and once with 1X PBS with 0.1% Tween for 5 minutes each, the slides were mounted on the slides with Prolong Diamond Anti-fade DAPI5 Mounting Media (Invitrogen- Molecular Probes, Eugene, OR) to visualize cell nucleic. For negative controls, primary antibody was omitted. A confocal microscope system (Nikon A1R+si Confocal Microscope) was used to capture high-resolution image. Experiments were repeated in duplicates for four antibodies.

**Table 1. Antibodies**

| **Antibody** | **Catalog No.** | **Company** | **Concentration** |
|---|---|---|---|
| CD3 | ab699 | Abcam (Cambridge, MA, USA) | 1:50 |
| CD4 | 553043 | BD Pharmigen (Franklin Lakes, NJ) | 1:50 |
| CD45 | sc-70690 | Santa Cruz (Santa Cruz Biotechnology, Santa Cruz, CA) | 1:50 |
| Alexa Fluor 488 goat anti-mouse IgG (H+L) | A11001 | Invitrogen. Life Technologies (Invitrogen, Eugene, OR) | 1:1000 |
| Alexa Fluor 488 goat anti-rat IgG (H+L) | A11006 | Invitrogen. Life Technologies (Invitrogen, Eugene, OR) | 1:1000 |
| Alexa Fluor 594 goat anti-mouse IgG (H+L) | A11032 | Invitrogen. Life Technologies | 1:1000 |
| Alexa Fluor 594 goat anti-rat IgG (H+L) | A11007 | Invitrogen. Life Technologies | 1:1000 |

### Outcome measures:

- Iris congestion (0-3), Anterior chamber cells (0-4), Flare (0-3), Vitreous haze (0-4).
- Combined anterior segment inflammatory score = sum of iris congestion, anterior chamber cells and flare.
- Histology and immunohistochemical staining: H&E, CD3, CD4, CD45.

### Results

### Inflammatory scores

Table 2 shows the mean combined anterior segment inflammatory scores. One day after subconjunctival injection, the combined anterior segment inflammatory score was significantly lower in the liposomal prednisolone phosphate (PP) group than in the controls (5.4±1.5 vs 8.4±1.7, p=0.049), and was also significantly lower than the eye drops group (p=0.033) This difference persisted till 5 days after initial intervention, with both liposomal groups (2.6±2.1, p=0.019 and 3.3±2.5, p=0.024 in the liposomal PP and liposomal triamcinolone phosphate (TA) groups respectively) demonstrating significantly lower combined anterior segment inflammatory scores than controls (7.2±2.2). Liposomal PP achieved greater attenuation of rebound inflammation than controls on day 11, 3 days after a rechallenge of intravitreal TB antigen (4.7±2.6 vs 8.5±1.3, p=0.041). A single dose of subconjunctival liposomal PP or TA delivered sustained anti-inflammatory for 2 weeks post treatment, similar to daily Pred forte eyedrops instilled 4 times a day for 2 weeks (5.0±2.8 and 5.0±1.0 for lipsomal PP and TA respectively, vs 4.6±1.3 for eye drops, p>0.05).

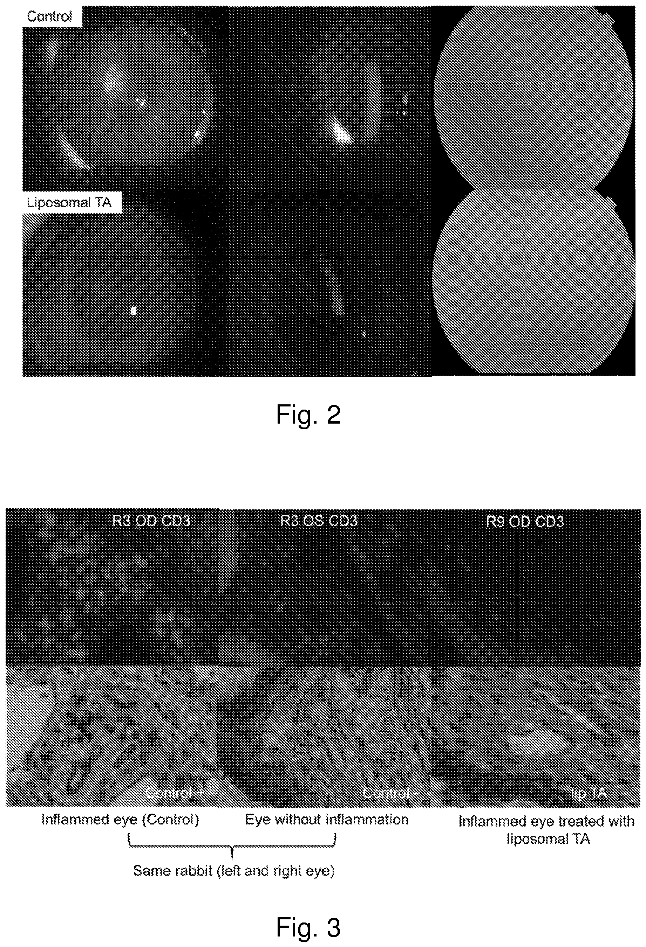

Figure 2 shows slit lamp and fundus photos at Day 11.

**Table 2. Inflammatory scores**

| **Day** | **Combined anterior segment inflammatory score** | | | | | |
|---|---|---|---|---|---|---|
| | Liposomal PP (n=5) | Liposomal TA (n=6) | Free PP (n=5) | Pred Forte 1% eyedrops (n=5) | Controls (n=5) | †P |
| 0 | **1st intravitreal induction** | | | | | |
| 1 | 9.4±0.5 | 9.7±0.5 | 8.6±0.5 | 9.0±1.0 | 9.6±0.5 | 0.08 |
| 3 | 9.4±0.5 | 9.7±0.5 | 8.0±0.7 | 9.0±1.4 | 9.0±1.4 | 0.35 |
| 3 | **Intervention** | | | | | |
| 4 | 5.4±1.5* | 6.5±1.9 | 6.0±0.7 | 8.0±1.4* | 8.4±1.7* | 0.02 |
| 8 | 2.6±2.1** | 3.3±2.5** | 3.2±0.4 | 6.0±0.7 | 7.2±2.2** | 0.002 |
| 8 | **2^{nd} intravitreal induction** | | | | | |
| 9 | 5.8±2.7 | 7.0±2.4 | 8.0±1.2 | 8.8±1.3 | 8.5±2.4 | 0.13 |
| 11 | 4.712.6*** | 5.5±2.3 | 7.0±2.3 | 6.4±0.9 | 8.5±1.3*** | 0.04 |
| 16 | 5.0±2.8 | 5.0±1.0 | 5.4±1.3 | 4.6±1.3 | 7.6±1.9 | 0.08 |
| 24 | 1.4±1.5 | 2.2±1.7 | 2.8±0.4 | 1.2±1.6 | 4.0±2.2 | 0.08 |
| 31 | 0.8±1.8 | 2.0±2.5 | 2.2±1.3 | 1.8±1.9 | 3.2±1.8 | 0.44 |

| | | | | | | |
|---|---|---|---|---|---|---|
| †p values from one-way ANOVA, comparing mean combined anterior segment inflammatory scores between groups. ^{∗}Pairwise comparison between liposomal PP with controls, p=0.049 and with eyedrops, p=0.033 after bonferroni correction for multiple comparisons. ^{∗∗} Pairwise comparison between liposomal PP with controls, p=0.007, and pairwise comparison between liposomal TA with controls, p=0.019 after bonferroni correction for multiple comparisons. ^{∗∗∗} Pairwise comparison between liposomal PP with controls, p=0.041 after bonferroni correction for multiple comparisons. | | | | | | |

### Effect of liposomal steroid on cataract formation and intraocular pressure (IOP):

### Cataract formation

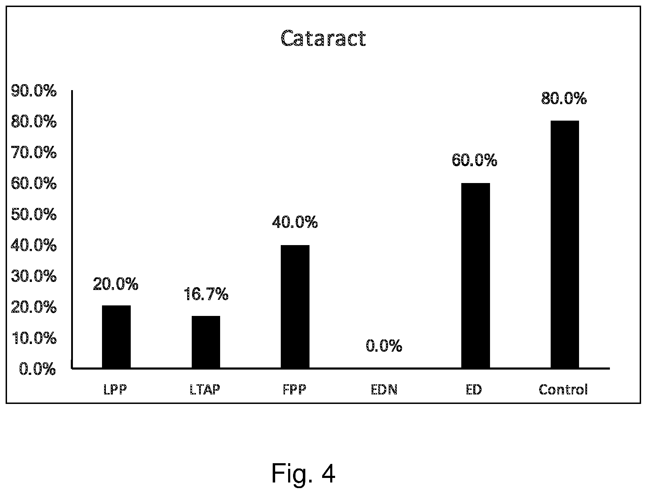

Overall, posterior subcapsular cataracts developed in 11 rabbits. No other subtype of cataracts was observed. There was no significant difference in the rate of cataract formation between treatment groups (p=0.185) but there was a trend towards higher rates in controls, eyedrops and subconjunctival free prednisolone phosphate groups. There were no significant differences in the severity of cataracts between groups. No cataracts were seen in fellow eyes administered with prednisolone acetate 1% eyedrops (EDN). The results are shown in Figure 4.

### Intraocular pressure (IOP)

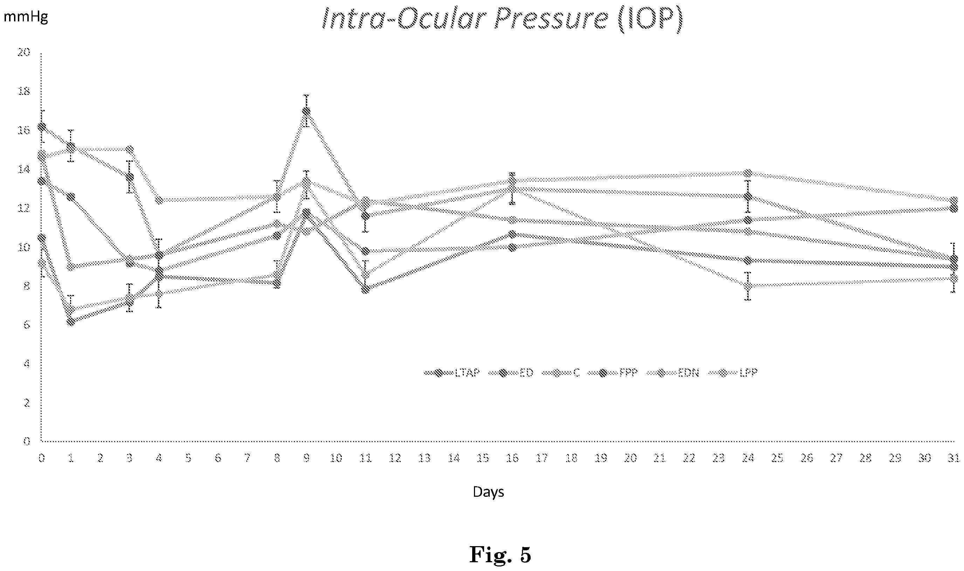

There were no significant differences in IOP between the treatment groups. No rabbit had raised IOP >21 at any point during the experiment. The results are shown in Figure 5.

### Histology:

Difference between control eyes (inflamed) and treated eyes is visible with HE staining and confirmed by immunostaining (Figure 3).

### Summary of results

In this study to assess the efficacy of subconjunctivally injected liposomal steroids for the treatment of experimental anterior uveitis, our key clinical findings were that: 1. These liposomal steroids demonstrate strong initial anti-inflammatory action and attenuation of rebound inflammation compared to eye drops; 2. A single dose of these liposomal steroids achieved sustained anti-inflammatory effect similar to 2 weeks of daily eye drop administration; 3. These liposomal steroids demonstrate sustained anti-inflammatory action compared to free steroid. At the same time, clinical efficacy corroborated well with histological analysis, and we observed co-localization of liposomes to the ciliary body suggesting targeted delivery of liposomes to inflammatory cells. Finally, we observed no adverse effects of liposomal steroids on IOP or cataract formation.

### References

Bloch-Michel E, Nussenblatt RB. International Uveitis Study Group recommendations for the evaluation of intraocular inflammatory disease. Am J Ophthalmol. 1987;103(2):234-235.
Ghosn CR, Li Y, Orilla WC, et al. Treatment of experimental anterior and intermediate uveitis by a dexamethasone intravitreal implant. Investigative ophthalmology & visual science. 2011;52(6):2917-2923.
Lobatto ME, Calcagno C, et al. Pharmaceutical development and preclinical evaluation of a GMP-grade anti-inflammatory nanotherapy. Nanomedicine: Nanotechnology, Biology, and Medicine. 2015; 11: 1133-1140
Nussenblatt RB, Palestine AG, Chan CC, Roberge F. Standardization of vitreal inflammatory activity in intermediate and posterior uveitis. Ophthalmology. 1985;92(4):467-471.

## Claims

1. Liposomes composed of non-charged vesicle-forming phospholipids, wherein said phospholipids comprise long-chain saturated fatty acids having an aliphatic tail of at least 13 carbon atoms, said liposomes optionally including not more than 10 mole percent of negatively charged vesicle-forming lipids and/or not more than 10 mole percent of PEGylated lipids, said liposomes having a selected mean particle diameter in the size range of 40-200 nm (as determined by Dynamic Light Scattering) and comprising a corticosteroid in water-soluble form, having a solubility at a temperature of 25°C of at least 10 g/l water or water buffered at neutral pH, for use in a method for the treatment of uveitis or conjunctivitis in a subject by subconjunctival administration at a dose of at most 5 mg corticosteroid, wherein said treatment has a treatment frequency of at most once per two weeks.

2. Liposomes for use according to claim 1 wherein said non-charged vesicle-forming phospholipids are selected from the group consisting of DiPaltmitoyl Phosphatidyl Choline (DPPC), Hydrogenated Soy Bean Phosphatidyl Choline (HSPC), DiStearoyl Phosphatidyl Choline (DSPC), Hydrogenated Egg Phosphatidyl Choline (HEPC) and combinations thereof.

3. Liposomes for use according to claim 1 or 2, wherein said negatively charged vesicle-forming lipids are selected from the group consisting of DiPalmitoyl Phosphatidyl Glycerol (DPPG), DiStearoyl Phosphatidyl Glycerol (DSPG) and a combination thereof.

4. Liposomes for use according to claim 1 or 2, wherein said liposomes are composed of:
- phospholipids selected from the group consisting of DPPC, HSPC, DSPC, HEPC and any combination thereof; and
- cholesterol.

5. Liposomes for use according to any one of claims 1-4, wherein said treatment comprises administering a single dose of said liposomes by subconjunctival injection.

6. Liposomes for use according to any one of claims 1-5, wherein said treatment has a treatment frequency of at most once per month.

7. Liposomes for use according to any one of claims 1-6, wherein said subject is a human.

8. Liposomes for use according to any one of claims 1-7, wherein said corticosteroid is selected from the group consisting of methylprednisolone disodium phosphate, methylprednisolone sodium succinate, prednisolone sodium phosphate, prednisolone sodium succinate and any combination thereof.

9. Liposomes for use according to any one of claims 1-8, wherein said corticosteroid is selected from the group consisting of prednisolone sodium phosphate, triamcinolone acetonide sodium phosphate, dexamethasone sodium phosphate, and any combination thereof.

## Patentansprüche

1. Liposome, zusammengesetzt aus nicht geladenen vesikelbildenden Phospholipiden, wobei die Phospholipide langkettige gesättigte Fettsäuren mit einem aliphatischen Schwanz von wenigstens 13 Kohlenstoffatomen umfassen, die Liposome optional einschließend nicht mehr als 10 Molprozent negativ geladene vesikelbildende Lipide und/oder nicht mehr als 10 Molprozent PEGylierte Lipide, die Liposomen mit einem ausgewählten mittleren Teilchendurchmesser in dem Größenbereich von 40-200 nm (bestimmt durch dynamische Lichtstreuung) und umfassend ein Corticosteroid in wasserlöslicher Form,
mit einer Löslichkeit bei einer Temperatur von 25°C von wenigstens 10 g/l Wasser oder Wasser gepuffert auf neutralen pH, zur Verwendung in einem Verfahren zur Behandlung von Uveitis oder Konjunktivitis in einem Subjekt durch subkonjunktivale Verabreichung in einer Dosis von höchstens 5 mg Corticosteroid, wobei die Behandlung eine Behandlungshäufigkeit von höchstens einmal pro zwei Wochen hat.

2. Liposomen zur Verwendung nach Anspruch 1, wobei die nicht geladenen vesikelbildenden Phospholipide ausgewählt sind aus der Gruppebestehend aus Dipalmitoylphosphatidylcholin (DPPC), hydriertem Sojabohnen Phosphatidylcholin (HSPC), Distearoylphosphatidylcholin (DSPC), hydriertem Ei-Phosphatidylcholin (HEPC) und Kombinationen davon.

3. Liposomen zur Verwendung nach Anspruch 1 oder 2, wobei die negativ geladenen vesikelbildenden Lipide ausgewählt sind aus der Gruppe bestehend aus Dipalmitoylphosphatidylglycerol (DPPG), Distearoylphosphatidylglycerol (DSPG) und einer Kombination davon.

4. Liposomen zur Verwendung nach Anspruch 1 oder 2, wobei die Liposomen zusammengesetzt sind aus:
- Phospholipiden, ausgewählt aus der Gruppe bestehend aus DPPC, HSPC, DSPC, HEPC und einer beliebigen Kombination davon; und
- Cholesterin.

5. Liposomen zur Verwendung nach einem der Ansprüche 1 - 4, wobei die Behandlung Verabreichen einer Einzeldosis der Liposomen durch subkonjunktivale Injektion umfasst.

6. Liposomen zur Verwendung nach einem der Ansprüche 1 - 5, wobei die Behandlung eine Behandlungshäufigkeit von höchstens einmal pro Monat hat.

7. Liposomen zur Verwendung nach einem der Ansprüche 1 - 6, wobei der Patient ein Mensch ist.

8. Liposomen zur Verwendung nach einem der Ansprüche 1 - 7, wobei das Kortikosteroid ausgewählt ist aus der Gruppe bestehend aus Methylprednisolondinatriumphosphat, Methylprednisolonnatriumsuccinat, Prednisolonnatriumphosphat, Prednisolonnatriumsuccinat und einer beliebigen Kombination davon.

9. Liposomen zur Verwendung nach einem der Ansprüche 1 - 8, wobei das Corticosteroid ausgewählt ist aus der Gruppe bestehend aus Prednisolonnatriumphosphat, Triamcinolonacetonidnatriumphosphat, Dexamethasonnatriumphosphat, und einer beliebigen Kombination davon.

## Revendications

1. Liposomes composés de phospholipides formant des vésicules non chargées, où lesdits phospholipides comprennent des acides gras saturés à longue chaîne ayant une queue aliphatique d'au moins 13 atomes de carbone, lesdits liposomes incluant éventuellement pas plus de 10 moles pour cent de lipides formant des vésicules chargées négativement et/ou pas plus de 10 moles pour cent de lipides PEGylés, lesdits liposomes ayant un diamètre de particule moyen choisi dans la gamme de tailles de 40-200 nm (comme déterminé par diffusion dynamique de la lumière) et comprenant un corticostéroïde sous forme soluble dans l'eau, ayant une solubilité à une température de 25°C d'au moins 10 g/l d'eau ou d'eau tamponnée à pH neutre, destinés à être utilisés dans un procédé pour le traitement de l'uvéite ou de la conjonctivite chez un sujet par administration sous-conjonctivale à une dose d'au plus 5 mg de corticostéroïde, où ledit traitement a une fréquence de traitement d'au plus une fois toutes les deux semaines.

2. Liposomes destinés à être utilisés selon la revendication 1, où lesdits phospholipides formant des vésicules non chargées sont choisis dans le groupe consistant en la dipalmitoylphosphatidylcholine (DPPC), la phosphatidylcholine de soja hydrogénée (HSPC), la distéaroylphosphatidyl-choline (DSPC), la phosphatidylcholine d'oeuf hydrogénée (HEPC) et leurs combinaisons.

3. Liposomes destinés à être utilisés selon la revendication 1 ou 2, où lesdits lipides formant des vésicules chargées négativement sont choisis dans le groupe consistant en le dipalmitoylphosphatidylglycérol (DPPG), le distéaroylphosphatidylglycérol (DSPC) et une combinaison de ceux-ci.

4. Liposomes destinés à être utilisés selon la revendication 1 ou 2, où lesdits liposomes sont composés de:
- phospholipides choisis dans le groupe consistant en DPPC, HSPC, DSPC, HEPC et toute combinaison de celles-ci; et
- cholestérol.

5. Liposomes destinés à être utilisés selon l'une quelconque des revendications 1 à 4, où ledit traitement comprend l'administration d'une dose unique desdits liposomes par injection sous-conjonctivale.

6. Liposomes destinés à être utilisés selon l'une quelconque des revendications 1 à 5, où ledit traitement a une fréquence de traitement d'au plus une fois par mois.

7. Liposomes destinés à être utilisés selon l'une quelconque des revendications 1 à 6, où ledit sujet est un humain.

8. Liposomes destinés à être utilisés selon l'une quelconque des revendications 1 à 7, où ledit corticostéroïde est choisi dans le groupe consistant en le phosphate disodique de méthylprednisolone, le succinate sodique de méthylprednisolone, le phosphate sodique de prednisolone, le succinate sodique de prednisolone et toute combinaison de ceux-ci.

9. Liposomes destinés à être utilisés selon l'une quelconque des revendications 1 à 8, où ledit corticostéroïde est choisi dans le groupe consistant en le phosphate sodique de prednisolone, le phosphate sodique d'acétonide de triamcinolone, le phosphate sodique de dexaméthasone et toute combinaison de ceux-ci.
